(19) Europäisches Patentamt
European Patent Office
Office européen des brevets

(11) Veröffentlichungsnummer: **0 469 462 B1**

(12) # EUROPÄISCHE PATENTSCHRIFT

(45) Veröffentlichungstag der Patentschrift: **11.10.95**

(51) Int. Cl.[6]: **C07D 251/42**

(21) Anmeldenummer: **91112470.9**

(22) Anmeldetag: **25.07.91**

Die Akte enthält technische Angaben, die nach dem Eingang der Anmeldung eingereicht wurden und die nicht in dieser Patentschrift enthalten sind.

(54) **2-Amino-(fluoralkoxy-1,3,5-triazine) und Verfahren zu ihrer Herstellung.**

(30) Priorität: **03.08.90 DE 4024761**

(43) Veröffentlichungstag der Anmeldung:
**05.02.92 Patentblatt 92/06**

(45) Bekanntmachung des Hinweises auf die Patenterteilung:
**11.10.95 Patentblatt 95/41**

(84) Benannte Vertragsstaaten:
**AT BE CH DE ES FR GB IT LI NL**

(56) Entgegenhaltungen:
**EP-A- 0 103 543**
**EP-A- 0 108 708**
**EP-A- 0 169 815**
**CA-A- 1 223 591**
**FR-A- 2 590 570**

**CHEMICAL ABSTRACTS, Band 112, Nr. 9, 26. Februar 1990, Columbus, Ohio, USA. MORISHIGE, JIRO et al.: "Synergistic herbicides containing a triazine and an acetanilide or a dinitroaniline", Seite 327, Spalte 1, Zusammenfassung-Nr. 72315k.**

**CHEMICAL ABSTRACTS, Band 109, Nr. 11, 12. September 1988, Columbus, Ohio, USA. CIBA LTD. "Herbicidal compositions comprising a N-phenylsulfonyl-N-(pyrimidin-2-yl- or 1,3,5-triazin-2-yl)urea and a benzaldoxime ether as antidote", Seite 262, Spalte 1, Zusammenfassung-Nr. 88185s.**

(73) Patentinhaber: **BASF Aktiengesellschaft**
**Carl-Bosch-Strasse 38**
**D-67063 Ludwigshafen (DE)**

(72) Erfinder: **Hamprecht, Gerhard, Dr.**
**Rote-Turm-Strasse 28**
**W-6940 Weinheim (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die Erfindung betrifft neue 2-Amino-(fluoralkoxy-1,3,5-triazine) der allgemeinen Formel I

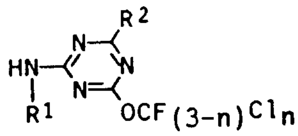

I,

in der

$R^1$ Wasserstoff, $C_1$-$C_4$-Alkyl, $C_3$-$C_6$-Alkenyl- oder $C_3$-$C_4$-Alkinyl

$R^2$ Wasserstoff, Halogen, $C_1$-$C_4$-Halogenalkyl, Trifluor- bzw. Chlordifluormethoxy und

n 0 oder 1

bedeuten.

Weiterhin betrifft die Erfindung ein Verfahren zur Herstellung der 1,3,5-Triazine I durch Umsetzung von Fluoralkoxy-2-halogen-1,3,5-triazinen mit Aminen.

Die 1,3,5-Triazine I sind wertvolle Zwischenprodukte für organische Synthesen, insbesondere für die Herstellung von Pflanzenschutzmitteln, z.B. herbizid wirksamen Sulfonylharnstoffderivaten.

EP-A-169 815 beschreibt $OCF_2Br$ substituierte Pyrimidine

In der Literatur finden sich vielfältige Hinweise, die aufgrund ähnlicher elektronischer Eigenschaften Fluoralkyl- und Fluoralkoxygruppen mit Halogenen gleichsetzen. pKa-Messungen (Proc. Natl. Acad. Sci. USA 134, 1207 (1960), J. Am. Chem. Soc. 83, 4860, (1961)) belegen, daß beispielsweise Fluoralkoxygruppen induktiv Elektronen ziehen, umgekehrt aufgrund ihrer Resonanzfähigkeit aber auch als Elektronendonor fungieren. Über alle Effekte gemessen ist die Trifluormethoxygruppe sogar stärker desaktivierend als die Halogene, so daß man auch von "Super-Halogenen" spricht (J. Am. Chem. Soc. 83, 4860, 1961). Dies gilt gleichermaßen für ihre Substituierbarkeit durch Nucleophile. Chemical Abstracts 87, 53396 belegt beispielsweise, daß 2,4-Bis(trichlormethyl)-6-trifluormethyl-1,3,5-triazin beim Rühren in Benzol mit basischen Aminen beide Halogenalkylgruppen austauscht. Die Fähigkeit des Trifluormethoxyrestes als Austrittsgruppe zu fungieren wird beispielsweise auch in der Zuckerchemie benutzt (CA. 105, 115325; 107, 96978).

Vor diesem Hintergrund ist verständlich, daß bisher noch kein Verfahren zur Herstellung von 2-Aminofluoralkoxy-1,3,5-triazinen bekannt wurde. Lediglich in der Reihe der Pyrimidine wurden 4-Difluormethoxy-2-halogen substituierte Vertreter zunächst mit dem giftigen Methylmercaptan in die 2-Methylthio-pyrimidinverbindungen umgewandelt, dann zu den 2-Methylsulfonylderivaten oxidiert und dann mit Amin nucleophil wieder verdrängt (US-A 4 542 216).

Es wurde nun gefunden, daß man die neuen 2-Amino-(fluoralkoxy-1,3,5-triazine) der allgemeinen Formel I,

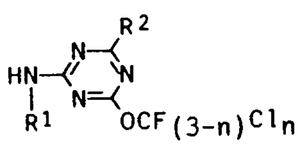

I,

in der

$R^1$, $R^2$ und n die vorgenannte Bedeutung besitzen, vorteilhaft erhält, wenn man 2-Halogen-1,3,5-triazine der Formel II,

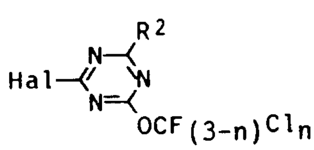

II,

in der Hal für Fluor, Chlor, Brom oder Jod steht und $R^2$ und n die vorgenannte Bedeutung besitzen, mit einem Amin der Formel III,

H-NH-$R^1$ III,

in der $R^1$ die vorgenannte Bedeutung hat, umsetzt.

Die Umsetzung kann für den Fall der Verwendung von 2,4-Difluor-6-trifluormethoxy-1,3,5-triazin und Ammoniak durch folgendes Schema beschrieben werden:

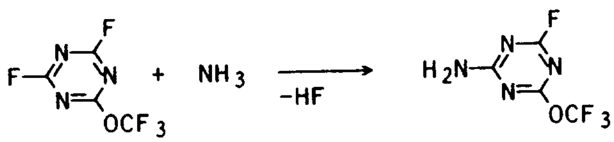


Das Verfahren liefert auf einfachem und wirtschaftlichem Weg neue 2-Aminofluoralkoxy-1,3,5-triazine in hoher Ausbeute und Reinheit. Entgegen der Erwartung werden Fluoralkoxygruppen unter den Reaktionsbedingungen nicht substituiert. Im Hinblick auf den Stand der Technik sind alle diese vorteilhaften Eigenschaften überraschend.

Bevorzugte Endstoffe I und dementsprechend bevorzugte Ausgangsstoffe II sind solche, in deren Formeln $R^1$ Wasserstoff, $C_1$-$C_4$-Alkyl wie Methyl, Ethyl, n-Propyl, i-Propyl, n-Butyl, sek-Butyl, i-Butyl, tert.-Butyl; $C_3$-$C_4$-Alkenyl wie 2-Propenyl, 2-Methylethenyl, 2-Butenyl, 3-Butenyl, 1-Methyl-2-propenyl oder 2-Methyl-2-propenyl; $C_3$-$C_4$-Alkinyl wie Propargyl, 2-Butinyl, 3-Butinyl oder 1-Methyl-2-propinyl; $R^2$ Wasserstoff, Fluor, Chlor, Brom, Trichlormethyl, Dichlorfluormethyl, Chlordifluormethyl, Trifluormethyl, 1,1-Dichlor-2,2,2-trifluorethyl, 1,1,2,2,2-Pentafluorethyl oder 1,1,2,2,2-Pentachlorethyl, ferner einen Trifluor- bzw. Chlordifluormethoxyrest und n 0 oder 1 bedeuten.

Unter den Aminen, welche eingesetzt werden können, sollen die folgenden erwähnt werden: Ammoniak, Methylamin, Ethylamin, n-Propylamin, Isopropylamin, n-Butylamin, Isobutylamin, sek. Butylamin, tert.-Butylamin, 2-Propenylamin, 2-Methylethenylamin, 2-Butenylamin, 3-Butenylamin, 1-Methyl-2-propenylamin, 2-Methyl-2-propenylamin, Propargylamin, 2-Butinylamin, 3-Butinylamin und 1-Methyl-2-propinylamin.

Die 2-Halogen-1,3,5-triazine II können in einem aprotisch polaren Lösungsmittel mit den Aminen III bei einer Temperatur von -80 bis 40 °C umgesetzt werden, wobei man das Amin III entweder in einem Überschuß, bezogen auf II, einsetzt oder eine organische Hilfsbase verwendet.

Die Umsetzung des 2-Halogen-1,3,5-triazins II mit dem Amin III kann in Abwesenheit oder vorteilhaft in Gegenwart eines Lösungsmittels vorgenommen werden. Als Lösungsmittel sind insbesondere die nachfolgend aufgeführten geeignet:

Ether wie Methyl-tert.-butylether` Diethylether, Ethylpropylether, n-Butylethylether, Di-n-butylether, Diisobutylether, Diisoamylether, Diisopropylether, Cyclohexylmethylether, Tetrahydrofuran, 1,2-Dimethoxyethan, Diethylenglykoldimethylether und Anisol, Ester wie Ethylacetat, n-Butylacetat und Isobutylacetat sowie chlorierte Kohlenwasserstoffe wie Methylenchlorid, 1,1,2,2-Tetrachlorethan, 1,1-Dichlorethylen, 1,2-Dichlorethan, Chlorbenzol, 1,2-Dichlorbenzol und 1-Chlornaphthalin und Gemische dieser Lösungsmittel.

Zweckmäßig verwendet man das Lösungsmittel in einer Menge von 100 bis 2000 Gew.-%, vorzugsweise 400 bis 1200 Gew.%, bezogen auf den Ausgangsstoff II.

Vorteilhaft gibt man 1,8 bis 2,5, insbesondere 1,95 bis 2,2 Molequivalent des Amins III, bezogen auf den Ausgangsstoff II, innerhalb 0,5 bis 2 Stunden zu einer Mischung von Ausgangsstoff II in einem der vorgenannten Lösungsmittel bei (-80) bis 40 °C, vorzugsweise -70 bis 25 °C, rührt bis zur Vervollständigung der Reaktion nach (ca. 3 Stunden) und läßt dann zur Aufarbeitung auf 25 °C erwärmen.

Setzt man nur ungefähr stöchiometrische Mengen des Amins III ein, so verwendet man zweckmäßig zusätzlich eine organische Hilfsbase, um den entstehenden Halogenwasserstoff abzufangen. Als Hilfsbase eignen sich dazu übliche organische Basen wie Trimethylamin, Triethylamin, N-Ethyl-diisopropylamin, Triisopropylamin, N,N-Dimethylanilin, N,N-Dimethylcyclohexylamin, N-Methylpyrrolidin, Pyridin, Chinolin, $\alpha$-, $\beta$- oder $\gamma$-Picolin, 2,4- und 2,6-Lutidin und Triethylendiamin. Im allgemeinen sind Zusätze von 0,9 bis 1,1 Equivalenten der Hilfsbase, bezogen auf den Ausgangsstoff II, ausreichend.

Die Reaktion kann drucklos oder unter Druck, kontinuierlich oder diskontinuierlich durchgeführt werden.

Die Aufarbeitung kann in üblicher Weise erfolgen, z.B. extrahiert man das Umsetzungsgemisch mit Wasser zur Entfernung der Salze, trocknet und reinigt die organische Phase, z.B. durch Chromatographie. Man kann jedoch auch direkt die organische Phase einengen und den Rückstand mit einem Lösungsmittel verrühren.

Im Hinblick auf ihre Weiterverarbeitung zu herbiziden Wirkstoffen, z.B. Sulfonylharnstoffderivaten, sind folgende 1,3,5-Triazine der Formel I besonders bevorzugt:
2-Amino-4-chlor-6-trifluormethoxy-1,3,5-triazin,
2-Amino-4-fluor-6-trifluormethoxy-1,3,5-triazin,
2-Amino-4-chlor-6-chlordifluormethoxy-1,3,5-triazin,
2-Amino-4-fluor-6-chlordifluormethoxy-1,3,5-triazin,
2-Amino-4,6-bis-trifluormethoxy-1,3,5-triazin,
2-Amino-4,6-bis-chlordifluormethoxy-1,3,5-triazin,
2-Amino-4-trifluormethoxy-6-trifluormethyl-1,3,5-triazin,
2-Amino-4-chlordifluormethoxy-6-trifluormethyl-1,3,5-triazin,
2-Methylamino-4-chlor-6-trifluormethoxy-1,3,5-triazin,
2-Methylamino-4-fluor-6-trifluormethoxy-1,3,5-triazin,
2-Methylamino-4-chlor-6-chlordifluormethoxy-1,3,5-triazin,
2-Methylamino-4-fluor-6-chlordifluormethoxy-1,3,5-triazin,
2-Methylamino-4,6-bis-trifluormethoxy-1,3,5-triazin,
2-Methylamino-4,6-bis-chlordifluormethoxy-1,3,5-triazin,
2-Methylamino-4-trifluormethoxy-6-trifluormethyl-1,3,5-triazin,
2-Methylamino-4-chlordifluormethoxy-6-trifluormethyl-1,3,5-triazin
2-Allylamino-4-chlor-6-trifluormethoxy-1,3,5-triazin,
2-Allylamino-4-fluor-6-trifluormethoxy-1,3,5-triazin,
2-Allylamino-4-chlor-6-chlordifluormethoxy-1,3,5-triazin,
2-Allylamino-4-fluor-6-chlordifluormethoxy-1,3,5-triazin,
2-Allylamino-4,6-bis-trifluormethoxy-1,3,5-triazin,
2-Allylamino-4,6-bis-chlordifluormethoxy-1,3,5-triazin,
2-Allylamino-4-trifluormethoxy-6-trifluormethyl-1,3,5-triazin,
2-Allylamino-4-chlordifluormethoxy-6-trifluormethyl-1,3,5-triazin
Der Herstellung der als Ausgangsstoffe II benötigen 2-Halogen-1,3,5-triazine kann nach dem in der japanischen Offenlegungsschrift 17 039 ('63), Chemical Abstracts 60, 2986 d beschriebenen Verfahren in Tetrachlorkohlenstoff vorgenommen werden. Wesentlich vorteilhafter aber ist das in der zeitgleichen deutschen Anmeldung P 40 24 755 (O.Z. 0050/41798) beschriebene Verfahren, das durch folgendes Reaktionsschema dargestellt wird:

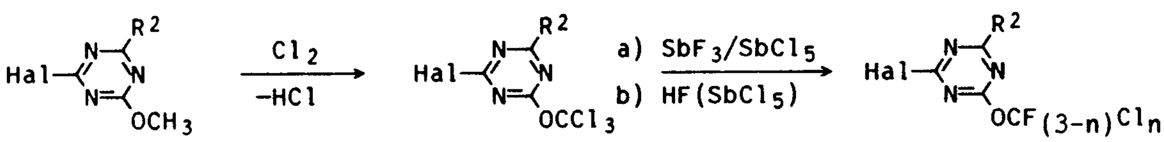


IV V II

Die Chlorierung des Methoxy-1,3,5-triazins IV mit Chlor zu dem Trichlormethoxy-1,3,5-triazin V führt man beispielsweise bei einer Temperatur von 100 bis 180 °C durch.

Als Chlorierungsmittel sind elementares Chlor oder Chlor abgebende Substanzen wie Sulfurylchlorid oder Phosphorpentachlorid geeignet. Chlor kann dabei auch in situ durch Oxidation von Chlorwasserstoffsäure, beispielsweise mit Wasserstoffsuperoxid hergestellt werden.

Die Chlorierung kann in Gegenwart eines inerten Lösungsmittels, beispielsweise einem chlorierten Kohlenwasserstoff wie Chlorbenzol, 1,2-, 1,3- oder 1,4-Dichlorbenzol, einer Nitroverbindung wie Nitrobenzol, einer Carbonsäure wie Essigsäure, Propionsäure, einem Säureanhydrid wie Essigsäureanhydrid, einem Säurechlorid wie Chloracetylchlorid, α-Chlorpropionsäurechlorid, α,α'-Dichlorpropionsäurechlorid, einem anorganischen Säurehalogenid wie Phosphortrichlorid oder Phosphoroxychlorid oder bevorzugt ohne Lösungsmittel in der Schmelze des Ausgangsstoffes durchgeführt werden.

Gegebenenfalls kann man die Reaktion durch Zugabe eines Radikalstarters beschleunigen; als solcher eignet sich die Bestrahlung mit Licht, vorzugsweise mit UV-Licht oder die Zugabe von α,α'-Azoisobutyronitril, zweckmäßig in einer Menge von 0,2 bis 7 Mol-%, bezogen auf den Ausgangsstoff. Man kann die Reaktion auch durch Zugabe eines Katalysators beschleunigen; als solcher eignet sich Phosphorpentachlorid, zweckmäßig in einer Menge von 0,5 bis 7 Mol-% bezogen auf den Ausgangsstoff IV. In diesem Fall legt man den Ausgangsstoff IV zusammen mit dem Katalysator vor und beginnt dann mit der Chlorierung. Statt des Phosphorpentachlorids kann man auch eine dieses unter den Reaktionsbedingungen bildende Ausgangskomponente, z.B. Phosphortrichlorid oder gelben Phosphor, zugeben und dann mit der Chlorierung beginnen.

Ausgangsstoff IV kann mit Chlor in annähernd stöchiometrischer Menge oder vorzugsweise im Überschuß, vorteilhaft mit 3,1 bis 11, insbesondere 3,3 bis 5 Mol Chlor je Equivalent Methoxy in dem Ausgangsstoff IV umgesetzt werden. Die Umsetzung kann bei einer Temperatur von 100 bis 180 °C, vorteilhaft von 120 bis 150 °C, drucklos oder unter Druck, kontinuerlich oder diskontinuierlich durchgeführt werden.

Chloriert man bei 1 bar, so werden zweckmäßig 3,3 bis 5 Mol Chlorgas, bezogen auf ein Equivalent Methoxy in dem Ausgangsstoff IV, eingesetzt, was einem Chlorumsatz von 91 bis 60 % entspricht. Durch geeignete apparative Maßnahmen, z B. durch Anwendung von mäßigem Überdruck, zweckmäßig 1 bis 10 bar, oder durch Verwendung einer Blasensäule, läßt sich der Chlorumsatz erhöhen. Vorteilhaft läßt man das Chlorgas möglichst lange mit der organischen Phase in Berührung kommen, indem diese beispielsweise stark gerührt wird oder das Chlorgas eine dicke Schicht der organischen Phase durchdringen muß. Die Reaktionszeit beträgt im allgemeinen etwa 0,5 bis 12 Stunden.

In einer bevorzugten Ausführungsform des Verfahrens geht man so vor, daß man innerhalb von 0,5 bis 12 Stunden, vorzugsweise 1 bis 10 Stunden die erforderliche Menge Chlorgas unter intensivem Rühren in den flüssigen Ausgangsstoff IV einleitet, wobei man zunächst bei einer Temperatur von 120 bis 130 °C beginnt und diese - gegebenenfalls unter Ausnutzung des exothermen Charakters der Reaktion - kontinuierlich steigert, so daß gegen Ende die Umsetzung bei einer Temperatur von 135 bis 150 °C durchgeführt wird. Bei größeren Reaktionsansätzen muß dem exothermen Charakter durch äußere Kühlung bzw. geeignete Dosierung der Chlormenge Rechnung getragen werden; mit dem Abflauen der Reaktion entfernt man das Kältebad und kann gegebenenfalls noch nacherhitzen.

Die Aufarbeitung und Isolierung der Endstoffe kann in üblicher Weise erfolgen. Beispielsweise kann man aus der heißen organischen Phase mittels eines Inertgases Reste an Chlorwasserstoff, Chlor oder Katalysator austragen; dabei hinterbleibt in hoher Ausbeute ein bereits recht reines Rohprodukt. Durch Destillation oder Chromatographie kann es weiter gereinigt oder aber direkt für weitere Umsetzungen eingesetzt werden.

Die Umsetzung des Trichlormethoxy-1,3,5-triazins V mit einem Halogenaustauschmittel führt man beispielsweise bei einer Temperatur von 0 bis 180 °C durch.

Als Halogenaustauschmittel sind Antimontrifluorid in Gegenwart oder Abwesenheit katalytischer Mengen eines Antimon(V)salzes, z.B. Antimon(V)chlorid oder Fluorwasserstoff geeignet. Zweckmäßig verwendet man einen Überschuß von 1 bis 200, vorzugsweise 5 bis 25 Mol-% Antimontrifluorid pro Trichlormethylequivalent. Die Katalysatormenge an Antimon(V)salz beträgt zwischen 1 bis 20, vorzugsweise 5 bis 18 Mol-% pro Trichlormethylequivalent. Vorzugsweise dosiert man den Ausgangsstoff V bei 90 bis 130 °C zu der Mischung des Halogenaustauschmittels und erwärmt dann noch zwischen 10 und ca. 240 Minuten auf eine Temperatur zwischen 110 und 180 °C. Anschließend wird z.B. destillativ aufgearbeitet.

Man kann die Reaktion jedoch auch kontinuierlich führen, den Ausgangsstoff V bei 110 bis 180 °C innerhalb 10 bis ca. 240 Minuten zugeben und gleichzeitig unter vermindertem Druck den entstehenden niedriger siedenden Endstoff II abdestillieren. Spuren mitgerissener Antimonsalze lassen sich durch Extraktion mit konz. Salzsäure beseitigen.

Arbeitet man ohne Antimon(V)salz-Katalyse oder setzt nur geringe Mengen, z. B. 0,5 bis 5 Mol-% ein, und reduziert die Menge an Antimontrifluorid auf 60 bis 90 Mol-% pro Trichlormethylequivalent, so bleibt der Halogenaustausch auf der Chlordifluormethoxystufe stehen.

Anstelle von Antimontrifluorid kann der Halogenaustausch auch mit Fluorwasserstoff bei 0 bis 150 °C, vorzugsweise 40 bis 120 °C, durchgeführt werden. Hierzu versetzt man in einem Autoklaven den Ausgangsstoff V mit einem Überschuß von 300 bis 700, vorzugsweise 350 bis 400 Mol-% Fluorwasserstoff pro Trichlormethylequivalent und rührt 10 Minuten bis ca. 10 Stunden nach. Gegebenenfalls kann die Reaktion in gleicher Weise, wie für die Verwendung von Antimontrifluorid beschrieben, durch Zusatz eines Katalysators wie Antimonpentachlorid beschleunigt werden. Im allgemeinen ist eine Nachreaktionszeit von ca. 4 Stunden ausreichend. Nach dem Entspannen und der Entfernung flüchtiger Bestandteile wird wie beschrieben aufgearbeitet.

Die neuen 2-Amino-(fluoralkoxy-1,3,5-triazine) I sind wertvolle Zwischenprodukte für die Herstellung von Pflanzenschutzmitteln. Nach dem Verfahren der zeitgleichen deutschen Patentanmeldung P 40 24 754 (O.Z. 0050/41800) können sie durch Umsetzung von beispielsweise 2-Amino-4-fluor-6-trifluormethoxy-1,3,5-triazin mit Methanol in das entsprechende 2-Amino-6-methoxy-4-trifluormethoxy-1,3,5-triazin umgewandelt werden, das mit 2-Carbomethoxy-benzolsulfonylisocyanat zu herbiziden Sulfonylharnstoffen reagiert. Sie können aber auch direkt mit dem genannten Isocyanat zu herbiziden Sulfonylharnstoffen umgesetzt werden.

Beispiele

I. Herstellbeispiele für die Vorprodukte (vgl. die zeitgleiche deutsche Anmeldung P 40 24 755 (O.Z. 0050/41798).

Beispiel I.1

2,4-Difluor-6-trichlormethoxy-1,3,5-triazin

In eine Mischung von 300 g (2,041 mol) 2,4-Difluor-6-methoxy-1,3,5-triazin und 0,3 g α,α'-Azoisobutyronitril leitete man bei 130 °C und unter UV-Bestrahlung einen Strom von Chlorgas so ein, daß sich während 2 Stunden eine Temperatur von 140 bis 145 °C einstellte. Nach NMR-spektroskopischer Kontrolle des Reaktionsverlaufs wurde unter äußerer Beheizung noch weitere 3 Stunden bei 135 bis 140 °C mit Chlor begast.

Nach dem Absaugen von ausgefallenem Niederschlag und Destillation des Filtrats im Vakuum erhielt man 444 g (87 % d. Th.) der Titelverbindung vom Sdp. 40 bis 46 °C/0,3 mbar.

Beispiel I.2

2,4-Difluor-6-trifluormethoxy-1,3,5-triazin

Zu einer Mischung von 187,4 g (1,048 mol) Antimontrifluorid und 35,2 g (0,117 mol) Antimonpentachlorid wurden die Hälfte von 210 g (0,838 mol) 2,4-Difluor-6-trichlormethoxy-1,3,5-triazin zunächst bei 110 °C unter Rühren so zugegeben, daß sich anfangs eine Temperatur von 125 °C einstellte; mit dem sich einstellenden Rückfluß mußte bei weiterer Zugabe außen beheizt werden. Es wurde eine Stunde bei 125 bis 130 °C gerührt und eine bei 100 bis 105 °C siedende Fraktion über eine 25-cm-Füllkörperkolonne abdestilliert. Nach dem Abklingen der Reaktion wurde die restliche Hälfte der Trichlormethoxyverbindung innerhalb 30 Minuten zugetropft und die bei 100 bis 105 °C übergehende Fraktion kontinuerlich abdestilliert. Die Gesamtreaktionszeit betrug 3 Stunden. Man erhielt 134,4 g (79,8 % d. Th.) der Titelverbindung, mit $n_D^{24}$ = 1.3650.

Beispiel I.3

6-Chlordifluormethoxy-2,4-difluor-1,3,5-triazin

210 g (0,838 mol) 2,4-Difluor-6-trichlormethoxy-1,3,5-triazin wurden innerhalb 10 Minuten unter Rühren bei 110 °C zu 110 g (0,615 mol) Antimontrifluorid gegeben. Nach Zugabe von 3/4 von 9,38 g (0,0313 mol) Antimonpentachlorid wurde auf 145 °C erwärmt und 1 Stunde gerührt. Restlicher Katalysator wurde zugegeben und das Reaktionsgemisch nochmals 2 Stunden gerührt, wobei als niedersiedende Fraktion über eine 30-cm-Füllkörperkolonne zwischen 95 bis 105 °C 20 g (11,8 % d. Th.) 2,4-Difluor-6-trifluormethoxy-1,3,5-triazin erhalten wurde. Der Destillationsrückstand wurde ohne Kolonne destilliert und ergab 94,8 g (52 % d. Th.) der Titelverbindung vom Sdp. 125 bis 130 °C; $n_D^{24}$ = 1.4042.

Beispiel I.4

2,4-Dichlor-6-trifluormethoxy-1,3,5-triazin

Zu einer Mischung von 40,9 g (0,229 mol) Antimontrifluorid und 7,03 g (0,0234 mol) Antimonpentachlorid wurden 52 g (0,183 mol) 2,4-Dichlor-6-trichlormethoxy-1,3,5-triazin innerhalb 5 Minuten unter Rühren bei 90 °C zugegeben, wobei sich die Temperatur bis auf 180 °C erhöhte. Es wurde noch 20 Minuten bei 170 bis 180 °C nachgerührt und dann das Rohprodukt bei 90 bis 103 °C/70 mbar abdestilliert. Durch nochmalige Destillation erhielt man 32,3 g (75,5 % d. Th.) der Titelverbindung vom Sdp. 165 bis 173 °C.

II. Herstellung der erfindungsgemäßen Verbindungen I

Beispiel II.1

2-Amino-4-fluor-6-trifluormethoxy-1,3,5-triazin

4,4 g (0,259 mol) Ammoniakgas wurden innerhalb 45 Minuten bei -70 bis -65 ° C unter Rühren in eine Mischung von 26,0 g (0,1293 mol) 2,4-Difluor-6-trifluormethoxy-1,3,5-triazin in 100 ml Tetrahydrofuran eingeleitet. Es wurde 2 Stunden bei -70 ° C und über Nacht unter Erwärmung bis auf 22 ° C gerührt. Nach dem Einengen im Vakuum wurde der Rückstand mit Wasser verrührt, abgesaugt und gewaschen. Nach dem Trocknen erhielt man 22 g (85,9 % d. Th.) der Titelverbindung vom Fp. 138 bis 139 ° C.

Beispiel II.2

2,4-Bismethylamino-6-trifluormethoxy-1,3,5-triazin und 2-Methylamino-4-fluor-6-trifluormethoxy-1,3,5-triazin

5,9 g (0,189 mol) Methylamin wurden bei -70 ° C innerhalb 30 Minuten unter Rühren in eine Mischung von 19,0 g (0,0945 mol) 2,4-Difluor-6-trifluormethoxy-1,3,5-triazin in 100 ml Diethylether eingegast. Es wurde 2 Stunden bei -70 ° C und über Nacht unter Erwärmung bis auf 22 ° C gerührt. Das Reaktionsgemisch wurde im Vakuum eingeengt, in Methylenchlorid aufgenommen und mit Wasser gewaschen. Nach dem Trocknen wurde über eine Kieselgelsäule fraktioniert chromatographiert, wobei man in den ersten beiden Fraktionen 5,0 g (25 % d. Th.) 2-Methylamino-4-fluor-6-trifluormethoxy-1,3,5-triazin vom Fp. 68 -72 ° C erhielt. In den weiteren Fraktionen 4 bis 7 isolierte man 10,7 g (51 % d. Th.) des schwerer löslichen 2,4-Bismethylamino-6-trifluormethoxy-1,3,5-triazins vom Fp. 150 bis 152 ° C.

Beispiel II.3

2-Amino-4-chlordifluormethoxy-6-fluor-1,3,5-triazin und 2,4-Diamino-6-chlordifluormethoxy-1,3,5-triazin

7,8 g (0,46 mol) Ammoniak wurden innerhalb 45 Minuten unter Rühren bei -70 ° C in eine Mischung von 50,0 g (0,23 mol) 2,4-Difluor-6-chlordifluormethoxy-1,3,5-triazin in 150 ml Tetrahydrofuran eingeleitet. Es wurde 2 Stunden bei -70 ° C und über Nacht unter Erwärmung bis auf 22 ° C gerührt. Das Reaktionsgemisch wurde im Vakuum eingeengt, mit Wasser gewaschen und getrocknet. Anschließend wurde das Reaktionsprodukt mit Methylenchlorid auf eine Kieselgelsäule geschlämmt und mit gleichem Lösungsmittel eluiert. In den Fraktionen 1 bis 8 erhielt man 21,5 g (43,6 % d. Th.) 2-Amino-4-fluor-6-chlordifluormethoxy-1,3,5-triazin vom Fp. 131 bis 133 ° C.

Durch Nachspülen mit Essigester isolierte man dann in den Fraktionen 9 bis 14 das schwerer lösliche 2,4-Diamino-6-chlordifluormethoxy-1,3,5-triazin (11,2 g, 23 % d. Th.) vom Fp. 114 ° C.

Beispiel II.4

2-Chlordifluormethoxy-4-fluor-6-methylaminol-1,3,5-triazin und 2,4-Bismethylamino-6-chlordifluormethoxy-1,3,5-triazin

5,2 g (0,166 mol) Methylamin wurden innerhalb 20 Minuten unter Rühren bei -70 ° C in eine Mischung von 18,1 g (0,083 mol) 4-Difluorchlormethoxy-2,6-difluor-1,3,5-triazin und Lösungsmittel eingeleitet. Es wurde 2 Stunden bei -70 ° C und über Nacht unter Erwärmung bis auf 22 ° C gerührt. Das Reaktionsgemisch wurde im Vakuum eingeengt, in Methylenchlorid aufgenommen, mit Wasser gewaschen und getrocknet. Nach Chromatographie über Kieselgel erhielt man in den ersten Fraktionen 5,5 g (29 % d. Th.) 2-Chlordifluormethoxy-4-fluor-6-methylamino-1,3,5-triazin vom Fp. 62 bis 64 ° C. Im Nachlauf isolierte man 8,7 g (44 % d. Th.) 2,4-Bismethylamino-6-chlordifluormethoxy-1,3,5-triazin vom Fp. 118 bis 120 ° C.

III. Umsetzung der 1,3,5-Triazine zu herbiziden Sulfonylharnstoffderivaten

Beispiel III.1

2-Amino-4-methoxy-6-trifluormethoxy-1,3,5-triazin

9,1 g (0,0505 mol) 30 %iges Natriummethylat wurden bei 0 °C innerhalb 15 Minuten unter Rühren zu einer Mischung von 10 g (0,0505 mol) 2-Amino-4-fluor-6-trifluormethoxy-1,3,5-triazin in 100 ml Methanol gegeben. Nach einer Stunde Rühren bei 0 °C wurde im Vakuum eingeengt, in Methylenchlorid aufgenommen und mit Wasser extrahiert. Nach dem Trocknen und Einengen erhielt man 10,5 g (99 % d. Th.) der Titelverbindung vom Fp. 96 bis 101 °C.

Beispiel III.2

2-Amino-4-chlordifluormethoxy-6-methoxy-1,3,5-triazin

8,4 g (0,0466 mol) 30 %iges Natriummethylat wurden bei 0 °C innerhalb 15 Minuten unter Rühren zu einer Mischung von 10 g (0,0466 mol) 2-Amino-4-chlordifluormethoxy-6-fluor-1,3,5-triazin in 100 ml Methanol gegeben. Nach einer Stunde Rühren bei 0 °C wurde im Vakuum eingeengt, in Methylenchlorid aufgenommen und mit Wasser extrahiert. Nach dem Trocknen und Einengen erhielt man 10,4 g (98,5 % d. Th.) der Titelverbindung vom Fp. 109 bis 111 °C.

Beispiel III.3

2-Amino-4-ethoxy-6-trifluormethoxy-1,3,5-triazin

2,3 g (0,093 mol) 97 % Natriumhydrid wurden portionsweise bei 20 bis 35 °C zu 300 ml Ethanol gegeben und 15 Minuten bis zur Lösung gerührt. Bei 0 °C wurden dann unter Rühren 18,5 g (0,093 mol) 2-Amino-4-fluor-6-trifluormethoxy-1,3,5-triazin innerhalb 10 Minuten zugegeben, 1 Stunde bei 0 °C und anschließend über Nacht bei 22 °C gerührt. Nach dem Einengen im Vakuum wurde der Rückstand in Methylenchlorid aufgenommen, mit Wasser extrahiert und getrocknet. Nach dem Einengen erhielt man 17,9 g (85,9 % d. Th.) der Titelverbindung vom Fp. 69 bis 71 °C.

Beispiel III.4

2-Amino-4-chloridfluormethoxy-6-ethoxy-1,3,5-triazin

1,2 g (0,0466 mol) 97 % Natriumhydrid wurden bei 20 bis 35 °C portionsweise zu 150 ml Ethanol gegeben und 15 Minuten bis zur Lösung gerührt. Anschließend wurde bei 0 °C unter Rühren 10,0 g (0,046 mol) 2-Amino-4-chlordifluormethoxy-6-fluor-1,3,5-triazin zugegeben, dann 1 Stunde bei 0 °C und über Nacht bei 22 °C gerührt. Nach dem Einengen im Vakuum wurde der Rückstand in Methylenchlorid aufgenommen, mit Wasser extrahiert und getrocknet. Nach dem Einengen erhielt man 10,6 g (94,6 % d. Th.) der Titelverbindung vom Fp. 63 bis 65 °C.

Beispiel III.5

2-Amino-4-methylamino-6-trifluormethoxy-1,3,5-triazin

3,5 g (0,111 mol) Methylamin wurden bei 0 °C innerhalb 20 Minuten unter Rühren in eine Lösung von 11 g (0,055 mol) 2-Amino-4-fluor-6-trifluormethoxy-1,3,5-triazin in 150 ml Tetrahydrofuran eingegast. Es wurde eine Stunde bei 0 °C und über Nacht bei 22 °C gerührt. Das Reaktionsgemisch wurde im Vakuum eingeengt, mit Wasser verrührt und getrocknet. Man erhielt 10,8 g (93,1 % d. Th.) der Titelverbindung vom Fp. 155 bis 157 °C (Zers.).

Beispiel III.6

2-Amino-4-chlordifluormethoxy-6-methylamino-1,3,5-triazin

2,9 g (0,0932 mol) Methylamin wurden innerhalb 20 Minuten unter Rühren bei 0 °C in eine Lösung von 10 g (0,0466 mol) 2-Amino-4-chlordifluormethoxy-6-fluor-1,3,5-triazin in 150 ml Diethylether eingegast. Es wurde eine Stunde bei 0 °C und über Nacht bei 22 °C gerührt. Nach dem Waschen mit Wasser, Trocknen und Einengen erhielt man 9,4 g (89,5 % d. Th.) der Titelverbindung vom Fp. 143 °C (Zers.).

Beispiel III.7

2-Amino-4-dimethylamino-6-trifluormethoxy-1,3,5-triazin

5,0 g (0,111 mol) Dimethylamin wurden innerhalb 20 Minuten unter Rühren bei 0 °C in eine Lösung von 11 g (0,055 mol) 2-Amino-4-fluor-6-trifluormethoxy-1,3,5-triazin in 150 ml Tetrahydrofuran eingegast. Es wurde eine Stunde bei 0 °C und über Nacht bei 22 °C gerührt. Nach dem Einengen, Waschen mit Wasser und Trocknen erhielt man 9,9 g (80,7 % d. Th.) der Titelverbindung vom Fp. 114 bis 118 °C (Zers.).

Beispiel III.8

2-Amino-4-chlordifluormethoxy-6-dimethylamino-1,3,5-triazin

4,2 g (0,093 mol) Dimethylamin wurden innerhalb 20 Minuten unter Rühren bei 0 °C in eine Lösung von 10 g (0,0466 mol) 2-Amino-4-chlordifluormethoxy-6-fluor-1,3,5-triazin in 150 ml Diethylether eingeleitet. Es wurde eine Stunde bei 0 °C und über Nacht bei 22 °C gerührt. Nach dem Waschen mit Wasser, Trocknen und Einengen erhielt man 9,8 g (87,8 % d. Th.) der Titelverbindung vom Fp. 130 bis 133 °C (Zers.).

Beispiel III.9

2-(((4-Methoxy-6-trifluormethoxy-1,3,5-triazin-2-yl)aminocarbonyl)aminosulfonyl)-benzoesäuremethylester

3,6 g (0,015 mol) 2-Carbomethoxy-benzolsulfonylisocyanat in 4 ml 1,2-Dichlorethan wurden innerhalb 5 Minuten unter Rühren bei 22 °C zu einer Mischung von 3,15 g (0,015 mol) 2-Amino-4-methoxy-6-trifluormethoxy-1,3,5-triazin in 150 ml 1,2-Dichlorethan gegeben und 12 Stunden bei 22 °C gerührt. Das Reaktionsgemisch wurde im Vakuum eingeengt und mit Methyl-tert.-butylether/Petrolether 1 : 1 kristallisiert, abgesaugt und mit Petrolether gewaschen. Man erhielt 5,1 g (75,4 % d. Th.) der Titelverbindung vom Fp. 149 °C (Zers.).

Beispiel III.10

2-(((4-Methoxy-6-trifluormethoxy-1,3,5-triazin-2-yl)aminocarbonyl)aminosulfonyl)-benzoesäuremethylesternatriumsalz

1,8 g (0,004 mol) der Verbindung aus Beispiel III.9 wurden in 30 ml Methanol suspendiert und mit 0,72 g (0,004 mol) 30 %iger Natriummethylatlösung unter Rühren bei 10 bis 15 °C versetzt. Die klare Lösung wurde nach 10 Minuten Rühren im Vakuum eingeengt, wobei 1,9 g (100 % d. Th.) der Titelverbindung vom Fp. 118 °C (Zers.) anfielen.

Beispiel III.11

2-(((4-Methylamino-6-trifluormethoxy-1,3,5-triazin-2-yl)aminocarbonyl)-aminosulfonyl)-benzoesäureethylester

3,1 g (0,012 mol) 2-Carboethoxy-benzolsulfonylisocyanat in 3 ml Methylenchlorid wurden innerhalb 10 Minuten unter Rühren bei 22 °C zu einer Mischung von 2,5 g (0,012 mol) 2-Amino-4-methylamino-6-trifluormethoxy-1,3,5-triazin in 150 ml Methylenchlorid gegeben und 30 Stunden bei 22 °C gerührt. Das Reaktionsgemisch wurde im Vakuum eingeengt, mit Methyl-tert.-butylether verrührt und abgesaugt. Nach weiterem Waschen mit Methanol und Trocknen erhielt man 3,8 g (67,4 % d. Th.) der Titelverbindung vom Fp. 182 bis 184 °C (Zers.).

**Patentansprüche**

**Patentansprüche für folgende Vertragsstaaten : AT, BE, CH, DE, FR, GB, IT, LI, NL**

**1.** Substituierte 2-Amino-(fluoralkoxy-1,3,5-triazine) der allgemeinen Formel I,

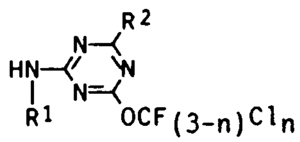

I,

in der

$R^1$ Wasserstoff, $C_1$-$C_4$-Alkyl, $C_3$-$C_6$-Alkenyl- oder $C_3$-$C_4$-Alkinyl;

$R^2$ Wasserstoff, Halogen, $C_1$-$C_4$-Halogenalkyl, einen Trifluor- bzw. Chlordifluormethoxy und

n 0 oder 1

bedeuten.

**2.** Verfahren zur Herstellung von 2-Amino-(fluoralkoxy-1,3,5-triazinen) der allgemeinen Formel I,

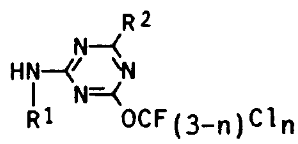

I,

in der

$R^1$ Wasserstoff, $C_1$-$C_4$-Alkyl, $C_3$-$C_6$-Alkenyl- oder $C_3$-$C_4$-Alkinyl;

$R^2$ Wasserstoff, Halogen, $C_1$-$C_4$-Halogenalkyl, einen Trifluor- bzw. Chlordifluormethoxyrest und

n 0 oder 1

bedeuten,

dadurch gekennzeichnet, daß man 2-Halogen-1,3,5-triazine der Formel II

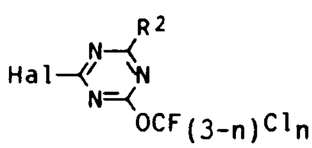

II,

in der Hal für Fluor, Chlor, Brom oder Jod steht und $R^2$ und n die vorgenannte Bedeutung besitzen, mit einem Amin der Formel III,

$H-NH-R^1$ III,

in der $R^1$ die vorgenannte Bedeutung hat, ggf. in Gegenwart einer organischen Hilfsbase, umsetzt.

**3.** Verfahren nach Anspruch 2, dadurch gekennzeichnet, daß man als Amin Ammoniak verwendet.

**4.** Verfahren nach Anspruch 2, dadurch gekennzeichnet, daß man als Amin Methylamin verwendet.

**5.** Verfahren nach den Ansprüchen 2 und 4, dadurch gekennzeichnet, daß man die Umsetzung bei einer Temperatur von -80 bis 40 °C durchführt.

**6.** 2-Amino-(fluoralkoxy-1,3,5-triazin) der Formel I, gemäß Anspruch 1, in der $R^1$ Wasserstoff, $R^2$ Fluor und n = 0 bedeuten.

**7.** 2-Amino-(fluoralkoxy-1,3,5-triazin) der Formel I, gemäß Anspruch 1, in der $R^1$ Wasserstoff, $R^2$ Fluor und n = 1 bedeuten.

**8.** 2-Amino-(fluoralkoxy-1,3,5-triazin) der Formel I, gemäß Anspruch 1, in der $R^1$ Methyl, $R^2$ Fluor und n = 0 bedeuten.

**9.** 2-Amino-(fluoralkoxy-1,3,5-triazin) der Formel I, gemäß Anspruch 1, in der $R^1$ Methyl, $R^2$ Fluor und n = 0 bedeuten.

**Patentansprüche für folgenden Vertragsstaat : ES**

**1.** Verfahren zur Herstellung von 2-Amino-(fluoralkoxy-1,3,5-triazinen) der allgemeinen Formel I,

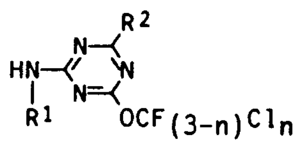

I,

in der
$R^1$ Wasserstoff, $C_1$-$C_4$-Alkyl, $C_3$-$C_6$-Alkenyl- oder $C_3$-$C_4$-Alkinyl;
$R^2$ Wasserstoff, Halogen, $C_1$-$C_4$-Halogenalkyl, einen Trifluor- bzw. Chlordifluormethoxyrest und
n 0 oder 1
bedeuten,
dadurch gekennzeichnet, daß man 2-Halogen-1,3,5-triazine der Formel II

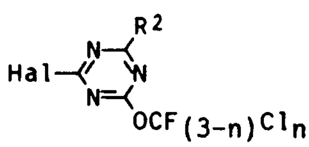

II,

in der Hal für Fluor, Chlor, Brom oder Jod steht und $R^2$ und n die vorgenannte Bedeutung besitzen, mit einem Amin der Formel III,

H-NH-$R^1$ III,

in der $R^1$ die vorgenannte Bedeutung hat, ggf. in Gegenwart einer organischen Hilfsbase, umsetzt.

**2.** Verfahren nach Anspruch 1, dadurch gekennzeichnet, daß man als Amin Ammoniak verwendet.

**3.** Verfahren nach Anspruch 1, dadurch gekennzeichnet, daß man als Amin Methylamin verwendet.

**4.** Verfahren nach den Ansprüchen 1 und 3, dadurch gekennzeichnet, daß man die Umsetzung bei einer Temperatur von -80 bis 40 ° C durchführt.

**Claims**

**Claims for the following Contracting States : AT, BE, CH, DE, FR, GB, IT, LI, NL**

1. A substituted 2-amino-(fluoroalkoxy-1,3,5-triazine) of the formula I

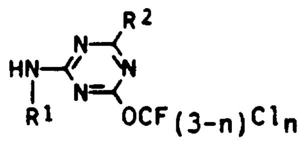

I

where

$R^1$ is hydrogen, $C_1$-$C_4$-alkyl, $C_3$-$C_6$-alkenyl or $C_3$-$C_4$-alkynyl;
$R^2$ is hydrogen, halogen, $C_1$-$C_4$-haloalkyl, trifluoromethoxy or chlorodifluoromethoxy and
n is 0 or 1.

2. A process for preparing a 2-amino-(fluoroalkoxy-1,3,5-triazine) of the formula I

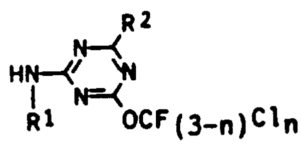

I

where

$R^1$ is hydrogen, $C_1$-$C_4$-alkyl, $C_3$-$C_6$-alkenyl or $C_3$-$C_4$-alkynyl;
$R^2$ is hydrogen, halogen, $C_1$-$C_4$-haloalkyl, trifluoromethoxy or chlorodifluoromethoxy and
n is 0 or 1,

which comprises reacting a 2-halo-1,3,5-triazine of the formula II

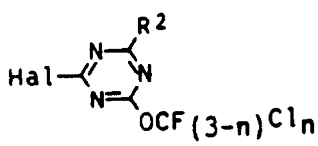

II

where Hal is fluorine, chlorine, bromine or iodine, and $R^2$ and n have the abovementioned meanings, with an amine of the formula III

$H\text{-}NH\text{-}R^1$ III

where $R^1$ has the abovementioned meaning, in the presence or absence of an additional organic base.

3. A process as claimed in claim 2, wherein ammonia is used as amine.

4. A process as claimed in claim 2, wherein methylamine is used as amine.

5. A process as claimed in claims 2 and 4, wherein the reaction is carried out at from -80 to 40 °C.

6. A 2-amino-(fluoroalkoxy-1,3,5-triazine) of the formula I as claimed in claim 1, where $R^1$ is hydrogen, $R^2$ is fluorine and n is 0.

7. A 2-amino-(fluoroalkoxy-1,3,5-triazine) of the formula I as claimed in claim 1, where $R^1$ is hydrogen, $R^2$ is fluorine and n is 1.

**8.** A 2-amino-(fluoroalkoxy-1,3,5-triazine) of the formula I as claimed in claim 1, where $R^1$ is methyl, $R^2$ is fluorine and n is 0.

**9.** A 2-amino-(fluoroalkoxy-1,3,5-triazine) of the formula I as claimed in claim 1, where $R^1$ is methyl, $R^2$ is fluorine and n is 0.

**Claims for the following Contracting State : ES**

**1.** A process for preparing a 2-amino-(fluoroalkoxy-1,3,5-triazine) of the formula I

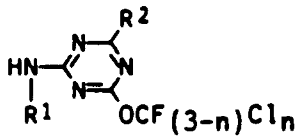

I

where
$R^1$ is hydrogen, $C_1$-$C_4$-alkyl, $C_3$-$C_6$-alkenyl or $C_3$-$C_4$-alkynyl;
$R^2$ is hydrogen, halogen, $C_1$-$C_4$-haloalkyl, trifluoromethoxy or chlorodifluoromethoxy and
n is 0 or 1,
which comprises reacting a 2-halo-1,3,5-triazine of the formula II

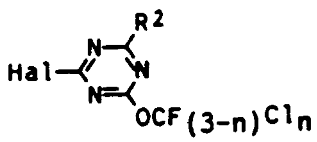

II

where Hal is fluorine, chlorine, bromine or iodine, and $R^2$ and n have the abovementioned meanings, with an amine of the formula III

H-NH-$R^1$ III

where $R^1$ has the abovementioned meaning, in the presence or absence of an additional organic base.

**2.** A process as claimed in claim 1, wherein ammonia is used as amine.

**3.** A process as claimed in claim 1, wherein methylamine is used as amine.

**4.** A process as claimed in claims 1 and 3, wherein the reaction is carried out at from -80 to 40 °C.

**Revendications**
**Revendications pour les Etats contractants suivants : AT, BE, CH, DE, FR, GB, IT, LI, NL**

**1.** 2-amino-(fluoralcoxy-1,3,5-triazines) substituées de formule générale I

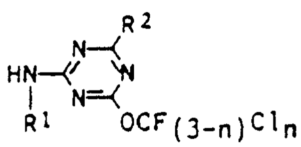

I,

dans laquelle
$R^1$ représente l'hydrogène, un groupe alkyle en C1-C4, alcényle en C3-C6 ou alcynyle en C3-C4 ;
$R^2$ représente l'hydrogène, un halogène, un groupe halogénoalkyle en C1-C4, un groupe trifluoro- ou

chlorodifluoro-méthoxy et
n est égal à 0 ou 1.

**2.** Procédé de préparation des 2-amino-(fluoralcoxy-1,3,5-triazines) de formule générale I

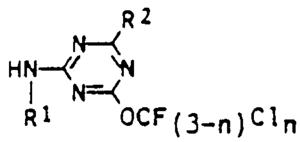

I,

dans laquelle
$R^1$ représente l'hydrogène, un groupe alkyle en C1-C4, alcényle en C3-C6 ou alcynyle en C3-C4 ;
$R^2$ représente l'hydrogène, un halogène, un groupe halogénoalkyle en C1-C4, un groupe trifluoro- ou chlorodifluoro-méthoxy et
n est égal à 0 ou 1,
caractérisé par le fait que l'on fait réagir des 2-halogéno-1,3,5-triazinesde formule II

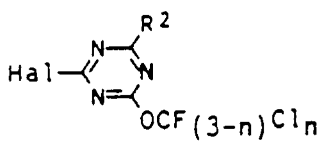

II,

dans laquelle Hal représente le fluor, le chlore, le brome ou l'iode et $R^2$ et n ont les significations indiquées ci-dessus, avec une amine de formule III

$H\text{-}NH\text{-}R^1$ III,

dans laquelle $R^1$ a les signications indiquées ci-dessus, éventuellement en présence d'une base organique auxiliaire.

**3.** Procédé selon la revendication 2, caractérisé en ce que l'amine utilisée est l'ammoniac.

**4.** Procédé selon la revendication 2, caractérisé en ce que l'amine utilisée est la méthylamine.

**5.** Procédé selon les revendications 2 et 4, caractérisé en ce que l'on effectue la réaction à une température allant de -80 à 40 °C.

**6.** 2-amino-(fluoralcoxy-1,3,5-triazine) de formule I selon la revendication 1 dans laquelle $R^1$ représente l'hydrogène, $R^2$ le fluor et n = 0.

**7.** 2-amino-(fluoralcoxy-1,3,5-triazine) de formule I selon la revendication 1 dans laquelle $R^1$ représente l'hydrogène, $R^2$ le fluor et n = 1.

**8.** 2-amino-(fluoralcoxy-1,3,5-triazine) de formule I selon la revendication 1 dans laquelle $R^1$ représente un groupe méthyle, $R^2$ le fluor et n = 0.

**9.** 2-amino-(fluoralcoxy-1,3,5-triazine) de formule I selon la revendication 1 dans laquelle $R^1$ représente un groupe méthyle, $R^2$ le fluor et n = 0.

**Revendications pour l'Etat contractant suivant : ES**

1. Procédé de préparation des 2-amino-(fluoralcoxy-1,3,5-triazines) de formule générale I

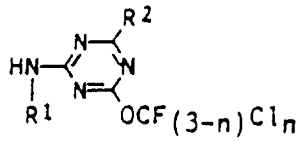

I,

dans laquelle
$R^1$ représente l'hydrogène, un groupe alkyle en C1-C4, alcényle en C3-C6 ou alcynyle en C3-C4 ;
$R^2$ représente l'hydrogène, un halogène, un groupe halogénoalkyle en C1-C4, un groupe trifluoro- ou chlorodifluoro-méthoxy et
n est égal à 0 ou 1,
caractérisé par le fait que l'on fait réagir des 2-halogéno-1,3,5-triazinesde formule II

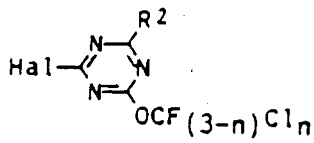

II,

dans laquelle Hal représente le fluor, le chlore, le brome ou l'iode et $R^2$ et n ont les significations indiquées ci-dessus, avec une amine de formule III

H-NH-$R^1$ III,

dans laquelle $R^1$ a les significations indiquées ci-dessus, éventuellement en présence d'une base organique auxiliaire.

2. Procédé selon la revendication 1, caractérisé en ce que l'amine utilisée est l'ammoniac.

3. Procédé selon la revendication 1, caractérisé en ce que l'amine utilisée est la méthylamine.

4. Procédé selon les revendications 1 et 3, caractérisé en ce que l'on effectue la réaction à une température allant de -80 à 40 ° C.